# EUROPEAN PATENT SPECIFICATION

(11) **EP 2 755 638 B1**
(45) Date of publication and mention of the grant of the patent: **01.06.2016**
(21) Application number: 12772421.9
(22) Date of filing: 14.09.2012
(51) Int. Cl.: A61K 9/16, A61K 9/20, A61K 9/28, A61K 9/48, A61K 9/50, A61K 31/485

(54) **TAMPER RESISTANT IMMEDIATE RELEASE FORMULATIONS**
MANIPULATIONSSICHERE FORMULIERUNGEN FÜR SOFORTIGE FREISETZUNG
FORMULATIONS À LIBÉRATION IMMÉDIATE RÉSISTANT AU DÉTOURNEMENT D'UTILISATION

(30) Priority: 16.09.2011 US 201161535758 P
(43) Date of publication of application: 23.07.2014
(73) Proprietor: Purdue Pharma LP, Stamford, CT 06901-3431 (US)
(72) Inventor: REILLY, Kevin, Kendall Park, NJ 08824 (US)
(74) Representative: Maiwald Patentanwalts GmbH
(86) International application number: PCT/IB2012/001916
(87) International publication number: WO 2013/038268

(56) References cited:
- EP-A1- 1 897 545
- WO-A1-95/20947
- WO-A1-2009/025859
- WO-A2-2009/023672
- WO-A2-2012/085657
- US-A1- 2004 131 671
- US-A1- 2007 224 129
- US-A1- 2008 063 725
- US-A1- 2008 152 595
- US-A1- 2008 254 112

## Description

### FIELD OF THE INVENTION

The present invention relates to the field of pharmaceutical dosage forms that are resistant to tampering and abuse.

### BACKGROUND

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Opioid agonist formulations intended for oral use are sometimes crushed or subject to extraction with solvents (e.g., ethanol) by drug abusers to provide the opioid contained therein for non-prescribed illicit use (e.g., nasal or parenteral administration).

There have previously been attempts in the art to control the abuse potential associated with opioid analgesics. For example, the combination of pentazocine and naloxone has been utilized in tablets available in the United States, commercially available as Talwin® Nx from Sanofi-Winthrop. Talwin® Nx contains pentazocine hydrochloride equivalent to 50 mg base and naloxone hydrochloride equivalent to 0.5 mg base. Talwin® Nx is indicated for the relief of moderate to severe pain. The amount of naloxone present in this combination has low activity when taken orally, and minimally interferes with the pharmacologic action of pentazocine. However, this amount of naloxone given parenterally has profound antagonistic action to narcotic analgesics. Thus, the inclusion of naloxone is intended to curb a form of misuse of oral pentazocine which occurs when the dosage form is solubilized and injected. Therefore, this dosage has lower potential for parenteral misuse than previous oral pentazocine formulations. A fixed combination therapy comprising tilidine (50 mg) and naloxone (4 mg) has been available in Germany for the management of severe pain since 1978 (Valoron® N, Goedecke). The rationale for the combination of these drugs is effective pain relief and the prevention of tilidine addiction through naloxone-induced antagonisms at the morphine receptor. A fixed combination of buprenorphine and naloxone was introduced in 1991 in New Zealand (Temgesic® Nx, Reckitt & Colman) for the treatment of pain.

WO 2009/023672 A2 relates to an abuse resistant oral pharmaceutical composition, comprising: a barrier layer, comprising a first polymer; and a diffusion layer, comprising a second polymer, substantially covering the barrier layer, wherein the diffusion layer is bonded to the barrier layer and comprises a drug that is substantially homogeneously distributed within the second polymer and diffuses from the diffusion layer within the gastrointestinal (GI) tract, wherein the pharmaceutical composition may optionally comprise an expansion layer comprising an expandable polymer and wherein the barrier layer substantially covers the expansion layer.

US 2007/0224129 A1 relates to a solid oral pharmaceutical formulation which is characterized in that it contains anti-misuse means, in that at least part of the AP it comprises is contained in coated microparticles for modified release of the AP, and in that the coated microparticles of AP have a coating layer (Ra) which assures the modified release of the AP and simultaneously imparts crushing resistance to the coated microparticles of AP so as to avoid misuse.

WO 2009/025859 A1 relates to a dosage form for sustained release of paliperidone, comprising at least a first component and second component, wherein the first component comprises at least one delay layer comprising a polymer, and the second component comprises non-coated Paliperidone and optionally comprises also coated Paliperidone; wherein the second component is located adjacent to the first component.

US 2008/0063725 A1 relates to an oral pharmaceutical form comprising microparticles of reservoir type, with modified release of at least one AP, not subject to dose dumping in the presence of alcohol which resists immediate AP dose dumping in the presence of alcohol, in particular in a large volume and, in addition, the composition and the structure of which make it possible to prevent misuse of the AP this form contains, especially due to anti-misuse means.

EP 1 897 545 A1 relates to tamper resistant solid oral extended release pharmaceutical dosage forms comprising an extended release matrix formulation.

US 2008/0152595 A1 relates to a pharmaceutical composition (e. g. , an oral solid pharmaceutical product) of any active drug substance susceptible to abuse, a gel forming polymer, a surfactant in sufficient amounts to cause nasal or mucosal irritation, and an agent in sufficient amounts to cause flushing, or other unpleasant peripheral vasodilatory effects, if the amount of the active drug subject to abuse is ingested in amounts exceeding the usual recommended therapeutic dose.

WO95/20947 relates to a tablet containing two or more layers comprising one or more drugs and one or more gelling agents, characterised in that the drug (s) and gelling agent(s) are contained in separate layers of the tablet.

WO 2012/085657 A2 relates to a solid oral dosage form comprising (a) an inert tamper resistant core; and (b) a coating surrounding the core, the coating comprising an active agent.

US 2004/0131671 A1 relates to a sustained release formulation of Acetaminophen and tramadol.

US 2008/0254112 A1 relates to a pharmaceutical active ingredient-containing formulation for oral administration which is coated with a single coating of a film-forming polymer, the coating comprising a mixture of at least two separating agents and no stabilizer.

There exists a need in the art for a dosage form containing a drug susceptible to abuse that is resistant to parenteral and/or nasal abuse. In the case of opioid analgesics, there exists a need for a tamper resistant formulation that does not solely rely upon the inclusion of an antagonist in the formulation to deter parenteral and/or nasal abuse.

### SUMMARY OF THE INVENTION

It is an object of certain embodiments of the present invention to provide an immediate release solid oral dosage form comprising an active agent (e.g., an opioid analgesic) which is tamper resistant.

It is an object of certain embodiments of the present invention to provide an immediate release solid oral dosage form comprising an active agent (e.g., an opioid analgesic) which is subject to less parenteral abuse than other dosage forms.

It is an object of certain embodiments of the present invention to provide an immediate release solid oral dosage form comprising an active agent (e.g., an opioid analgesic) which is subject to less intranasal abuse than other dosage forms.

It is a further object of certain embodiments of the present invention to provide an immediate release solid oral dosage form comprising an active agent (e.g., an opioid analgesic) which is subject to less diversion than other dosage forms.

It is a further object of certain embodiments of the present invention to treat a disease or condition (e.g., pain) in human patients by administering an immediate release solid oral dosage form as disclosed herein to a patient in need thereof.

It is a further object of certain embodiments of the present invention to provide a method of treating pain in human patients with an immediate release solid oral dosage form comprising an opioid analgesic while reducing the abuse potential of the dosage form.

It is a further object of certain embodiments of the present invention to provide a method of manufacturing an immediate release solid oral dosage form of an active agent (e.g., an opioid analgesic) as disclosed herein.

It is a further object of certain embodiments of the present invention to provide a use of a medicament (e.g., an opioid analgesic) in the manufacture of a tamper-resistant dosage form as disclosed herein for the treatment of a disease state (e.g., pain).

The above objects of the present invention and others can be achieved by the present invention, which in certain not claimed embodiments is directed to an immediate release solid oral dosage form comprising a plurality of particles, each particle comprising (i) a core comprising a gelling agent; (ii) an optional barrier layer surrounding the core; and (iii) an active layer comprising a drug susceptible to abuse surrounding the barrier layer.

In certain embodiments, the plurality of particles are contained within a pharmaceutically acceptable capsule. In certain other embodiments, the plurality of particles are compressed into a tablet. In certain embodiments, the capsule or tablet further contains a pharmaceutically acceptable diluent.

In certain embodiments, the present invention is directed to a unit dose of an immediate release solid oral dosage form, said unit dose comprising from about 2 to about 75 particles, each particle consisting of (i) a core comprising a gelling agent in the form of a compressed tablet; (ii) an active layer comprising a drug susceptible to abuse surrounding the core; and (iii) optionally an outer coating directly coated onto the final layered compressed core; wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C; and wherein the viscosity of the dosage form mixed with from 0.5 to 10 ml of an aqueous liquid is at least 10 mPa s (10 cP). In certain embodiments, the unit dose is contained within a pharmaceutically acceptable capsule. In certain other embodiments, the unit dose is a tablet formed from compression of the particles. In certain embodiments, the unit dose further comprises a pharmaceutically acceptable diluent.

In certain not claimed embodiments, the present invention is directed to a unit dose of an immediate release solid oral dosage form, said unit dose comprising from about 2 to about 75 particles, each particle comprising (i) a core comprising a gelling agent; (ii) a barrier layer surrounding the core; and (iii) an active layer comprising a drug susceptible to abuse surrounding the barrier layer on the core. In certain embodiments, the unit dose is contained within a pharmaceutically acceptable capsule. In certain other embodiments, the unit dose is a tablet formed from compression of the particles. In certain embodiments, the unit dose further comprises a pharmaceutically acceptable diluent.

In certain embodiments, the solid oral dosage form disclosed herein releases at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

In certain embodiments, the solid oral dosage form disclosed herein releases at least about 90% by weight, or at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

In other embodiments, the viscosity resulting from mixing a unit dose of the dosage form with from about 0.5 to about 10 ml of an aqueous liquid prevents the drug from being absorbed, or reduces the ability of the drug to be absorbed, by parenteral or nasal administration.

A process for preparing an immediate release solid oral dosage form comprising a plurality of particles comprises (i) preparing a plurality of cores, each core comprising a gelling agent; and (ii) applying an active layer comprising a drug susceptible to abuse so that it surrounds each core.

The process may be directed to preparing an immediate release solid oral dosage form in accordance with not claimed embodiments, comprising a plurality of particles, comprising (i) preparing a plurality of cores, each core comprising a gelling agent; (ii) applying a barrier layer so that it surrounds each core; and (iii) applying an active layer comprising a drug susceptible to abuse so that it surrounds the barrier layer on each core.

The process may further comprise containing the particles within a pharmaceutically acceptable capsule to form a unit dose. The process may further comprise compressing the particles into a tablet form to form a unit dose comprising from about 2 to about 75 particles.

The process may be directed to the preparation of a dosage form as disclosed herein that releases at least about 85% by weight, or at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

The process may be directed to the preparation of a solid oral dosage form as disclosed herein that releases at least about 90% by weight, or at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

The process may be directed to the preparation of a dosage form as disclosed herein wherein the viscosity resulting from mixing a unit dose of the dosage form (crushed or uncrushed) with from about 0.5 to about 10 ml of an aqueous liquid prevents the drug from being absorbed, or reduces the ability of the drug to be absorbed, by parenteral or nasal administration.

A method of treating a disease or condition (e.g., pain, diarrhea or constipation) comprises administering to a patient in need thereof an immediate release dosage form as disclosed herein.

In describing the present invention, the following terms are to be used as indicated below. As used herein, the singular forms "a," "an," and "the" include plural references unless the context clearly indicates otherwise. Thus, for example, reference to "an active agent" includes a single active agent as well as a mixture of two or more different active agents, and reference to a "gelling agent" includes a single gelling agent as well as a mixture of two or more different gelling agents, and the like.

As used herein, the terms "active agent," "active ingredient," "pharmaceutical agent," and "drug" refer to any material that is intended to produce a therapeutic, prophylactic, or other intended effect, whether or not approved by a government agency for that purpose. These terms with respect to specific agents include all pharmaceutically active forms of the agent, including the free base form of the agent, and all pharmaceutically acceptable salts, complexes, stereoisomers, crystalline forms, cocrystals, ether, esters, hydrates, solvates, and mixtures thereof, where the form is pharmaceutically active.

As used herein, the terms "therapeutically effective" refers to the amount of drug or the rate of drug administration needed to produce a desired therapeutic result.

As used herein, the terms "prophylactically effective" refers to the amount of drug or the rate of drug administration needed to produce a desired prophylactic result.

As used herein, the term "stereoisomers" is a general term for all isomers of individual molecules that differ only in the orientation of their atoms in space. It includes enantiomers and isomers of compounds with one or more chiral centers that are not mirror images of one another (diastereomers).

The term "enantiomer" or "enantiomeric" refers to a molecule that is non-superimposable on its mirror image and hence optically active wherein the enantiomer rotates the plane of polarized light in one direction by a certain degree, and its mirror image rotates the plane of polarized light by the same degree but in the opposite direction.

The term "chiral center" refers to a carbon atom to which four different groups are attached.

The term "racemic" refers to a mixture of enantiomers.

The term "resolution" refers to the separation or concentration or depletion of one of the two enantiomeric forms of a molecule.

The term "patient" means a subject, particularly a human, who has presented a clinical manifestation of a particular symptom or symptoms suggesting the need for treatment, who is treated preventatively or prophylactically for a condition, or who has been diagnosed with a condition to be treated. The term "subject" is inclusive of the definition of the term "patient" and does not exclude individuals who are entirely normal in all respects or with respect to a particular condition.

"Pharmaceutically acceptable salts" include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate and the like; amino acid salts such as arginate, asparaginate, glutamate and the like; metal salts such as sodium salt, potassium salt, cesium salt and the like; alkaline earth metals such as calcium salt, magnesium salt and the like; and organic amine salts such as triethylamine salt, pyridine salt, picoline salt, ethanolamine salt, triethanolamine salt, discyclohexylamine salt, N,N'-dibenzylethylenediamine salt and the like.

The term "ppm" as used herein means "parts per million". Regarding 14-hydroxycodeinone, "ppm" means parts per million of 14-hydroxycodeinone in a particular sample product. The 14-hydroxycodeinone level can be determined by any method known in the art, preferably by HPLC analysis using UV detection.

### BRIEF DESCRIPTION OF THE DRAWINGS

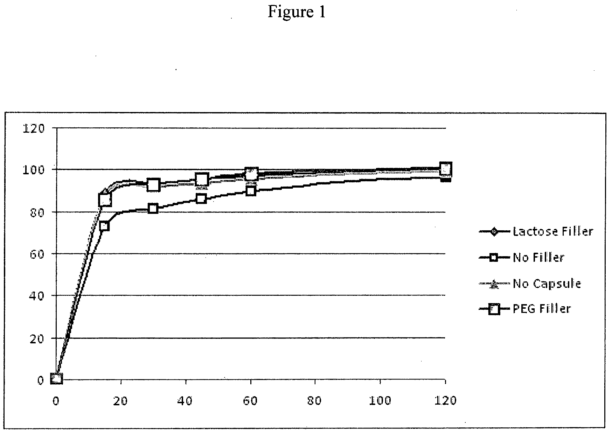

Figure 1 is a graphical depiction of the dissolution results of Example 6.

### DETAILED DESCRIPTION

Gelling agents have been contemplated for use in pharmaceutical formulations in order to deter the abuse of dosage forms containing a drug susceptible to abuse (e.g., an opioid analgesic). One form of abuse is the crushing of a controlled release dosage form in order to liberate the drug contained therein for illicit use such as parenteral administration or through absorption across a mucosal surface. When a dosage form having a gelling agent is crushed and then mixed with a solution, a viscosity is obtained which may inhibit the drug from being drawn into a needle, thereby hindering parenteral abuse. Similarly, when the crushed dosage form is applied to a mucosal surface (e.g., the nasal cavity) the composition will gel upon contact with mucosal moisture, thereby inhibiting absorption.

Controlled release dosage forms of drugs of abuse have received considerable attention in an attempt to develop tamper-resistant technologies as the crushing of the dosage form may liberate an amount of active agent normally intended for prolonged release (e.g., 12 to 24 hours)

Immediate release dosage forms are also the subject of abuse and present public safety issues when administered by other than the intended route. One problem to overcome in incorporating a gelling agent into an immediate release dosage form is controlled release characteristics that such an agent may impart to a dosage form when included in sufficient amounts to inhibit tampering.

In certain situations, an immediate release or controlled release dosage form can be abused without crushing, e.g., by contacting the intact dosage form with a liquid to dissolve the active agent contained therein. This can be a particular issue with intact dosage forms that are in particulate form, given the larger surface area and increased dissolution of such dosage forms.

Both controlled release and immediate release multiparticulate formulations may have formulation and pharmacokinetic issues such as (i) difficulty in manufacture, (ii) dose to dose variability in active agent, (iii) pharmacokinetic variability, (iv) variability due to administration with food, and (v) patient-to-patient variability which may be addressed by the present invention.

Immediate release and controlled release dosage forms play a vital role in the management of both acute and chronic conditions (e.g., pain management with opioid analgesics). Therefore, it is important to provide a tamper-resistant dosage form of a drug susceptible to abuse that may be utilized for either controlled or immediate release dosage forms to obtain a viable product that can provide effective plasma levels to a patient according to an intended release profile.

In certain other not claimed embodiments, the present invention is directed to an immediate release solid oral dosage form comprising a plurality of particles, each particle comprising:
(i) a core comprising a gelling agent;
(ii) a barrier layer surrounding the core; and
(iii) an active layer comprising a drug susceptible to abuse surrounding the barrier layer on the core;
wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C; and
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The present invention is directed to a unit dose of an immediate release oral dosage form comprising from about 2 to about 75 particles, each particle consisting of:
(i) a core comprising a gelling agent in the form of a compressed tablet; and
(ii) an active layer comprising a drug susceptible to abuse surrounding the core; and
(iii) optionally an outer coating directly coated onto the final layered compressed core;
wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C; and
wherein the viscosity resulting from mixing a unit dose of the dosage form with from about 0.5 to about 10 ml of an aqueous liquid prevents the drug from being absorbed, or reduces the ability of the drug to be absorbed, by parenteral or nasal administration and is at least 10 mPa s (10 cP).

In other not claimed embodiments, the present invention is directed to a unit dose of an immediate release oral dosage form comprising from about 2 to about 75 particles, each particle comprising:
(i) a core comprising a gelling agent;
(ii) a barrier layer surrounding the core; and
(iii) an active layer comprising a drug susceptible to abuse surrounding the barrier layer on the core;
wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C; and
wherein the viscosity resulting from mixing a unit dose of the dosage form with from about 0.5 to about 10 ml of an aqueous liquid prevents the drug from being absorbed, or reduces the ability of the drug to be absorbed, by parenteral or nasal administration.

In alternative not claimed embodiments, each particle of the immediate release dosage form may further comprise:
(iv) a second barrier layer encompassing the active layer; and
(v) a second active layer comprising a non-orally bioavailable opioid antagonist encompassing the second barrier layer.

In not claimed embodiments with a barrier layer, the material and/or amount of material utilized preferably will not substantially interfere with the release profile of the active agent from the dosage form. The material for the barrier layer can be, e.g., an acrylic polymer, a cellulosic polymer or a vinyl polymer. Preferred barrier layers include hydroxypropylmethylcellulose, polyvinyl alcohol, povidone or a mixture thereof.

In other embodiments, the plurality of particles are contained within a pharmaceutically acceptable capsule, preferably with the co-containment of an inert diluent. The diluent can be selected from saccharides (e.g., sucrose, dextrose, lactose, fructose, mannitol, and mixtures thereof), polyethylene glycols and cellulosic materials (e.g., microcrystalline cellulose).

The use of a barrier layer, co-containment with a diluent in a capsule, and selection of the number of particles in a unit dose, may all contribute to reducing agglomeration of the particles upon introduction to a dissolution medium either in vitro or in vivo (e.g., upon oral administration to a human subject) in order to maintain the immediate release profile of the dosage form.

In certain embodiments, the viscosity after mixing a dosage form (crushed or intact) with from about 0.5 to about 10 ml of an aqueous liquid is from about 10 cP to about 100 Cp; from about 25 cP to about 75 Cp; at least about 20 cP; at least about 40 cP or at least about 60 cP. In other embodiments, the viscosity after mixing a dosage form (crushed or intact) with from about 0.5 to about 10 ml of an aqueous liquid is at least about 10 cP, at least about 25 cP, at least about 75 Cp; at least about 100 cP; at least about 150 cP.

In certain embodiments, the weight amount of gelling agent contained in the dosage form of the present invention is not more than the weight amount of drug. In other embodiments, the weight amount of gelling agent contained in the immediate release dosage forms of the present invention is less than the weight amount of drug. In further embodiments, the weight amount of gelling agent contained in the immediate release dosage forms of the present invention is more than the weight amount of drug.

In certain embodiments, the dosage forms of the present invention contain a weight ratio of gelling agent to drug from about 5:1 to about 1:5; from about 3:1 to about 1:3; from about 1:1 to about 1:1.5; from about 1.5:1 to about 1:1; about 1:1.25; or about 1.25:1.

The gelling agent utilized in the dosage forms of the present invention can be selected from sugars, sugar derived alcohols (e.g., mannitol, sorbitol, and the like), starch and starch derivatives, cellulose derivatives (e.g., microcrystalline cellulose, sodium caboxymethyl cellulose, methylcellulose, ethyl cellulose, hydroxyethyl cellulose, hydroxypropyl cellulose, and hydroxypropyl methylcellulose), attapulgites, bentonites, dextrins, alginates, carrageenan, gum tragacanth, gum acacia, guar gum, xanthan gum, pectin, gelatin, kaolin, lecithin, magnesium aluminum silicate, carbomers, carbopols, polyvinylpyrrolidone, polyethylene glycol, polyethylene oxide, polyvinyl alcohol, silicon dioxide, surfactants, mixed surfactant/wetting agent systems, emulsifiers, other polymeric materials, and mixtures thereof. In certain embodiments, the gelling agent is xanthan gum. In other embodiments, the gelling agent is pectin. The pectin or pectic substances include purified or isolated pectates and crude natural pectin from sources such as apple, citrus or sugar beet residues which have been subjected, when necessary, to esterification or de-esterification (e.g., by alkali or enzymes). The pectins may also be derived from citrus fruits such as lime, lemon, grapefruit, and orange. In preferred embodiments, the gelling agent is selected from the group consisting of polyethylene oxide, hydroxypropylcellulose, hydroxyethylcellulose, hydroxypropylmethylcellulose, and mixtures thereof.

A unit dose of a dosage form of the present invention includes from about 2 to about 75 particles; from about 10 to about 50 particles; from about 15 to about 25 particles; or from about 10 to about 50 particles. In certain embodiments of the present invention, each unit dose in a batch contains the same amount of particles. Such embodiments may be preferable to typical multiparticulate dosage forms which may contain a greater number of particles and may not uniformly contain the same amount of particulates in each unit dose. Further, typical multiparticulate dosage forms may not have content uniformity for each individual particle.

In certain embodiments, each particle contains a sub-therapeutic amount of active agent but collectively in a unit dosage form contain a therapeutic amount of the active agent.

Certain embodiments of the present invention may address formulation and pharmacokinetic issues such as (i) difficulty in manufacture, (ii) dose to dose variability in active agent, (iii) pharmacokinetic variability, (iv) variability due to administration with food, and (v) patient-to-patient variability.

The particles of the present invention may have a mean diameter from about 0.1 mm to about 10 mm; from about 0.5 mm to about 8 mm; from about 1 mm to about 6 mm; or from about 2 mm to about 4 mm.

The core of the particles of the formulation is in the form of a compressed tablet.

The dosage forms of the present invention may comprise without limitation, from about 25% to about 99% core by weight; from about 50% to about 95% core by weight; or from about 65% to about 85% core by weight.

In certain not claimed embodiments, the barrier layer is applied to the core in an amount to provide a weight gain from about 1%(w/w) to about 10%(w/w); or from about 4%(w/w) to about 7%(w/w).

In certain embodiments, the immediate release dosage form of the present invention releases at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

In certain embodiments, the immediate release dosage form of the present invention releases at least about 90% by weight, at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

### Additional Excipients

The compressed cores of the present invention can include additional excipients in order to, e.g., aid manufacturing, provide additional tamper resistance, modify the release rate, or provide alcohol resistance.

The additional excipient may be at least one excipient selected from the group consisting of bulking agents, plasticizers, stabilizers, diluents, lubricants, binders, granulating aids, colorants, flavorants, and glidants.

In certain embodiments, the dosage form includes a polymer that can modify the release rate of the active agent contained therein. Examples of polymers that can be utilized to modify the release of the active agent include pharmaceutically acceptable cellulosic polymers, including but not limited to cellulose esters, cellulose diesters, cellulose triesters, cellulose ethers, cellulose ester-ethers, cellulose acylates, cellulose diacylates, cellulose triacylates, cellulose acetates, cellulose diacetates, cellulose triacetates, cellulose acetate propionates, cellulose acetate butyrates and mixtures thereof. Preferably, the cellulosic polymer is an alkyl cellulosic polymer such as methylcellulose or ethylcellulose.

In other embodiments of the present invention, the release modifying polymer is a pharmaceutically acceptable acrylic polymer selected without limitation from from acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid) (anhydride), methyl methacrylate, polymethacrylate, poly(methyl methacrylate), poly(methyl methacrylate) copolymer, polyacrylamide, aminoalkyl methacrylate copolymer, poly(methacrylic acid anhydride), glycidyl methacrylate copolymers, and mixtures of any of the foregoing.

Preferably, the acrylic polymer is a neutral acrylic polymer (e.g., Eudragit NE 30 D®, Eudragit NE 40 D® or Eudragit NM 30 D®), which can also provide crush-resistant characteristics to the dosage form.

Individual compressed cores can also include a film coating or barrier coating to enhance cosmetic appearance, reduce tackiness and/or provide stability. Examples of materials to be utilized as a film or barrier coat include hydroxypropylmethylcellulose, polyvinyl alcohol, lactose or a mixture thereof. The film or barrier coat can be (i) an coating directly coated onto a compressed core (not claimed), (ii) an outer coating directly coated onto a final layered compressed core, or (iii) an intermediate layer between any components of the dosage form (not claimed).

### Alcohol Resistance

The gelling agent and the optional release modifying agent can be selected in order to inhibit dose dumping of the active agent in the presence of alcohol. This characteristic is to prevent the dosage form from releasing the active agent at a rate faster than intended when alcohol is imbibed during residence of the dosage form in the gastrointestinal tract. Certain hydrophilic polymers (e.g., polyethylene oxide or methylcellulose) are suitable gelling agents that can provide alcohol resistance to the dosage form.

In certain embodiments, the amount of active agent (e.g. an opioid analgesic) released at 1 hour in 900 mL 0.1 N HCl (pH 1.5) with 40% EtOH using USP Apparatus II at 50 rpm is not more than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is less than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 25%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 10%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is not more than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is less than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 25%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 10%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is not more than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is less than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 25%(w/w) of the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 10%(w/w) of the amount of active agent released at 4 hour in 900 mL 0.1 N HCl (pH 1.5) with 0% EtOH using USP Apparatus II at 50 rpm.

### Tamper Resistance

In certain embodiments, the solid oral dosage form of the present invention demonstrates the tamper-resistant characteristic of not breaking or shattering when force is applied to it (by, for example, striking it with a hammer). Instead, the solid oral dosage form flattens without breaking or shattering. This characteristic makes it more difficult for the solid oral dosage form to be abused, by snorting the powder of a shattered tablet, chewing a tablet, or injecting a solution prepared from a shattered tablet. The inclusion of polyethylene oxide can provide tamper-resistant properties. The addition of neutral acrylic polymer also provides these properties.

In certain embodiments, the oral solid dosage form can be flattened without breaking, wherein the thickness of the dosage form after flattening corresponds to no more than about 60%, no more than about 50%, no more than about 40%, no more than about 30%, or no more than about 20% of the thickness of the dosage form before flattening.

In certain embodiments, the amount of active agent (e.g. an opioid analgesic) released at 0.5 hour from a flattened dosage form deviates no more than about 20%, no more than about 15%, or no more than about 10% points from a non-flattened dosage form as measured by an in-vitro dissolution in a USP Apparatus 1 (basket) at 100 rpm in 900 ml simulated gastric fluid without enzymes (SGF) at 37° C.

### Active Agents

In certain embodiments, the active agent used in the solid oral dosage form of the present invention is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, anti-pyretics, anti-inflammatory agents, androgens, local and general anesthetics, anti-addictive agents, anti-androgens, anti-arrhythmic agents, antiasthmatic agents, anti-cholinergic agents, anti-cholinesterase agents, anti-coagulants, anti-diabetic agents, anti-diarrheal agents, anti-diuretic, anti-emetic agents, pro-kinetic agents, anti-epileptic agents, anti-estrogens, anti-fungal agents, antihypertensive agents, anti-microbial agents, anti-migraine agents, anti-muscarinic agents, anti-neoplastic agents, anti-parasitic agents, anti-parkinson's agents, antiplatelet agents, anti-progestins, anti-schizophrenia agents, anti-thyroid agents, anti-tussives, anti-viral agents, atypical anti-depressants, azaspirodecanediones, barbiturates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof.

In certain embodiments, the active agent is an opioid agonist. In such embodiments, the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof. In certain embodiments, the opioid agonist is selected from the group consisting of codeine, fentanyl, hydromorphone, hydrocodone, oxycodone, dihydrocodeine, dihydromorphine, morphine, tramadol, oxymorphone, pharmaceutically acceptable salts thereof, and mixture thereof.

In certain embodiments, the opioid agonist is oxycodone or a pharmaceutically acceptable salt thereof in an amount, e.g., of about 2.5 mg, 5 mg, 7.5 mg or 10 mg.

In certain embodiments of the present invention, wherein the active agent is oxycodone hydrochloride, oxycodone hydrochloride is used having a 14-hydroxycodeinone level of less than about 25 ppm, less than about 15 ppm, less than about 10 ppm, less than about 5 ppm, less than about 2 ppm, less than about 1 ppm, less than about 0.5 ppm or less than about 0.25 ppm.

WO 2005/097801 A1, U.S. Pat. No. 7,129,248 B2 and US 2006/0173029 A1 describe a process for preparing oxycodone hydrochloride having low levels of 14-hydroxycodeinone.

In certain embodiments, the oral solid dosage form of the present invention comprises an active agent that is an opioid antagonist (with or without an opioid agonist). In such embodiments, the opioid antagonist is selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof.

In certain embodiments, the solid oral dosage form of the present invention comprises an active agent that is a non-opioid analgesic. In such embodiments, the non-opioid analgesic is a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.

In other embodiments, the present invention is directed to the dosage forms disclosed herein utilizing active agents such as benzodiazepines, barbiturates or amphetamines, their antagonists, or combinations thereof.

Benzodiazepines to be used in the present invention may be selected from alprazolam, bromazepam, chlordiazepoxide, clorazepate, diazepam, estazolam, flurazepam, halazepam, ketazolam, lorazepam, nitrazepam, oxazepam, prazepam, quazepam, temazepam, triazolam, and pharmaceutically acceptable salts, hydrates, and solvates and mixtures thereof. Benzodiazepine antagonists that can be used in the present invention include, but are not limited to, flumazenil and pharmaceutically acceptable salts, hydrates, and solvates.

Barbiturates to be used in the present invention include, but are not limited to, amobarbital, aprobarbotal, butabarbital, butalbital, methohexital, mephobarbital, metharbital, pentobarbital, phenobarbital, secobarbital and pharmaceutically acceptable salts, hydrates, and solvates mixtures thereof. Barbiturate antagonists that can be used in the present invention include, but are not limited to, amphetamines and pharmaceutically acceptable salts, hydrates, and solvates.

Stimulants to be used in the present invention include, but are not limited to, amphetamines, such as amphetamine, dextroamphetamine resin complex, dextroamphetamine, methamphetamine, methylphenidate and pharmaceutically acceptable salts, hydrates, and solvates and mixtures thereof. Stimulant antagonists that can be used in the present invention include, but are not limited to, benzodiazepines, and pharmaceutically acceptable salts, hydrates, and solvates as described herein.

### Methods of Manufacture

A process for preparing the immediate release oral dosage forms disclosed herein comprises:
(i) preparing a plurality of cores, each core comprising a gelling agent; and
(ii) applying an active layer comprising a drug susceptible to abuse so that it surrounds each core;
wherein the dosage form releases at least about 85% of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The process may be directed to the preparation of dosage forms in accordance with not claimed embodiments and comprises:
(i) preparing a plurality of cores, each core comprising a gelling agent;
(ii) applying a barrier layer to surround each core; and
(iii) applying an active layer comprising a drug susceptible to abuse so that it surrounds the barrier layer on each core;
wherein the dosage form releases at least about 85% of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The process may comprise:
(i) compressing (e.g., into a tablet) a plurality of cores comprising a gelling agent;
(ii) optionally applying a barrier layer surrounding each core (if directed to the preparation of dosage forms in accordance with not claimed embodiments); and
(iii) applying an active layer comprising a drug susceptible to abuse so that it surrounds the barrier layer;
wherein the dosage form releases at least about 85% of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The process comprises preparing an immediate release oral dosage form comprising from about 2 to about 75 particles prepared according to the invention.

The particles can be further processed into a unit dosage form, e.g., by containment in a capsule; containment in a sachet or paper; or compression into a tablet.

When the particles are contained in a capsule, certain embodiments include the co-containment of a diluent in the capsule along with the active agent particles. The incorporation of the diluent may serve to reduce agglomeration of the particles which can facilitate the dosage form maintaining the desired immediate release profile.

The diluent can be mixed with the particles and the mixture added to the capsule. Alternatively, the diluent can be added to the capsule before or after adding the active agent particles into the capsule.

In a preferred embodiment, the active agent particles are contained in the capsule and back-filled with the diluent.

The diluent can be as disclosed above and is preferably lactose or polyethylene glycol.

### Methods of Treatment

A method of treating a disease or condition comprises administering any of the solid oral dosage forms described herein to a patient in need thereof. The patient may be treated for pain, diarrhea, or constipation.

The method of treatment may comprise administering the solid oral dosage form described herein in combination with another pharmaceutical composition. The other pharmaceutical composition may be administered to treat the same condition or disease. The other pharmaceutical composition may also be administered to treat a different condition or disease.

The method of treatment of the present invention may further comprise monitoring the patient for how the patient metabolizes the active agent, or how the patient responds to the active agent. The method of treatment may further comprise altering the dose of the solid oral dosage form in response to said monitoring. Certain baseline measurements may be taken from the patient prior to administering the oral solid dosage form to the patient.

The following examples are set forth to assist in understanding the invention.

### Examples

### Example 1

### Preparation of Core Formulation

Cores comprising a gelling agent were prepared in accordance with Table 1:

**TABLE 1**

| Ingredient | mg / unit | Actual Amt Used (g) |
|---|---|---|
| Polyethylene Oxide (PEO 303) | 3.96 | 1980 |
| Magnesium Stearate | 0.04 | 20 |
| Total | 4.0 | 2000 |

The ingredients were processed according to the following procedure:
1. The polyethylene oxide was added to a polyethylene bag.
2. The magnesium stearate was added and bag blended with the polyethylene oxide for a sufficient time to obtain a substantially uniform blend.
3. The blend was compressed with a Kilian/IMA tablet press with 2 mm multi-tipped tooling with the following parameters:
   Target Tablet Weight: 4 mg
   Fill Depth/Setting: 3.00 - 3.05 mm
   Main Compression Force Target: 4 Kn
   Actual Compression Force: 3.5 - 4.5 Kn
   Actual Tablet Weights: 4.00 - 4.20 mg
   Actual Tablet Thickness: approximately 1.5 mm

### Reference Example 2

### Preparation of Barrier Layered Core Formulation

The cores of Example 1 were coated in accordance with Table 2:

**TABLE 2**

| Ingredient | mg / unit | Actual Amt Used (g) |
|---|---|---|
| Example 1 Cores | 4.00 | |
| Opadry | 0.24 | |
| DI Water* | | |
| **Total** | 4.24 | |

| | | |
|---|---|---|
| *DI Water is removed during processing and is not included in final weight | | |

The ingredients were processed according to the following procedure:
1. The DI water was added to a mixer and a vortex was created.
2. The Opadry was added to the DI water and mixed for about 1 hour.
3. The Vector VFC-3 Fluid Bed Processor, 4 liter chamber fitted with a Wurster Column was used to layer the solutions onto the substrate.
4. The processing parameters were as follows: The inlet temperature set point was about 65° to about 70° C; the product temperature was approximately 45° to about 50° C; the exhaust temperature was approximately 50° to about 55° C; the solution spray rate was approximately 4 grams per minute and the process air volume was approximately 70 cfm.
**5.** The Opadry solution was coated onto the cores of Example 1 to a weight gain of about 6%.

### Reference Example 3

### Preparation of Active Layered Core Formulation

The barrier layered cores of Example 2 were coated in accordance with Table 3:

**Table 3**

| Ingredient | mg/ barrier layered core | 20 barrier layered cores / unit | Actual Amt Used (g) |
|---|---|---|---|
| Barrier Layered Cores of Example 2 | 4.24 | 88.4 | 442 |
| Naltrexone HCl | 0.25 | 5 | 25 |
| Opadry | 0.318 | 6.36 | 31.8 |
| DI Water* | n/a* | n/a* | n/a* |
| **Total** | 4.988 | 99.76 | 498.8 |

| | | | |
|---|---|---|---|
| *DI Water is removed during processing and is not included in final weight | | | |

1. The DI water was added to a mixer and a vortex was created.
2. The Naltrexone HCl was added to the DI water and mixed until fully dispersed.
3. The Opadry was added to the Naltrexone HCl solution and mixed for about 1 hour.
4. The Vector VFC-3 Fluid Bed Processor, 4 liter chamber fitted with a Wurster Column was used to layer the solutions onto the sub-coated substrate.
5. The processing parameters were as follows: The product temperature set point was about 47° C; the product temperature was approximately 45° to about 50° C; the exhaust temperature was approximately 50° to about 55° C; the solution spray rate was approximately 4 grams per minute and the process air volume was approximately 80 cfm.
6. The Opadry/Naltrexone solution was coated onto the barrier layered cores of Example 2 to a weight gain of about 12.9%.

### Reference Example 4

### Preparation of Seal Layered Core Formulation

The active layered cores of Example 3 were coated in accordance with Table 4:

**Table 4**

| Ingredient | mg/ active layered core | 20 active layered cores / unit | Actual Amt Used (g) |
|---|---|---|---|
| Active Layered Cores of Ex. 3 | 5.066 | 101.32 | 450 |
| Opadry | 0.304 | 6.08 | 27.0 |
| DI Water* | n/a* | n/a* | n/a* |
| **Total** | 5.370 | 107.4 | 477.0 |

| | | | |
|---|---|---|---|
| *DI Water is removed during processing and is not included in final weight | | | |

1. The DI water was added to a mixer and a vortex was created.
2. The Opadry was added to the DI Water and mixed for about 1 hour.
3. The Vector VFC-3 Fluid Bed Processor, 4 liter chamber fitted with a Wurster Column was used to layer the solutions onto the sub-coated substrate.
4. The processing parameters were as follows: The product temperature set point was about 47° C; the product temperature was approximately 48°C; the exhaust temperature was approximately 45° to about 55° C; the solution spray rate was approximately 4 grams per minute and the process air volume was approximately 70 cfm.
5. The Opadry solution was coated onto the Active layered cores of Example 3 to a weight gain of 6.0%.

### Reference Example 5

### Active Layered Core Formulation Dissolution (Total Amount)

5 mg naltrexone HCl multiple tablet formulations were prepared in accordance with the present invention. The formulations were tested in SGF for the mg amount released at specific time points.
Formulation A was encapsulated with a lactose filler.
Formulation B was encapsulated without a filler.
Formulation C was directly added to the dissolution medium without a capsule.
Formulation D was encapsulated with a polyethylene glycol filler.

The results are set forth in Table 5 below:

**Table 5**

| | **Sample wt (mg) (capsule included)** | **Vessel** | **15.0 min** | **30.0 min** | **45.0 min** | **60.0 min** | **120.0 min** | **720.0 min** |
|---|---|---|---|---|---|---|---|---|
| Formulation A - 1 | 259.77 | A | 4.217 | 4.433 | 4.499 | 4.573 | 4.689 | 4.563 |
| Formulation A - 1 | 222.45 | A | 4.316 | 4.523 | 4.628 | 4.717 | 4.874 | 4.937 |
| Formulation A - 1 | 276.20 | A | 4.425 | 4.666 | 4.768 | 4.834 | 4.976 | 5.070 |
| **Mean (n=3)** | | | **4.32** | **4.54** | **4.63** | **4.71** | **4.85** | **4.86** |
| Formulation B -1 | 156.85 | A | 3.756 | 4.080 | 4.260 | 4.403 | 4.737 | 4.943 |
| Formulation B -1 | 160.37 | A | 3.593 | 4.059 | 4.272 | 4.475 | 4.836 | 5.055 |
| Formulation B -1 | 158.99 | A | 3.666 | 4.166 | 4.451 | 4.689 | 5.042 | 5.110 |
| **Mean(n=3)** | | | **3.67** | **4.10** | **4.33** | **4.52** | **4.87** | **5.04** |
| Formulation C -1 | 188.56 | B | 4.307 | 4.507 | 4.609 | 4.728 | 4.916 | 4.980 |
| Formulation C -1 | 186.30 | B | 4.376 | 4.542 | 4.626 | 4.708 | 4.870 | 4.913 |
| Formulation C -1 | 187.31 | B | 4.349 | 4.529 | 4.594 | 4.699 | 4.879 | 4.921 |
| **Mean** | | | **4.34** | **4.53** | **4.61** | **4.71** | **4.89** | **4.94** |
| Formulation D -1 | 402.02 | B | 4.021 | 4.478 | 4.628 | 4.733 | 4.885 | 4.907 |
| Formulation D -1 | 467.58 | B | 4.164 | 4.472 | 4.602 | * | * | 4.782 |
| Formulation D -1 | 425.72 | B | 4.374 | 4.669 | 4.824 | 4.879 | 4.974 | 4.993 |
| **Mean(n=3)** | | | **4.19** | **4.54** | **4.68** | **4.81** | **4.93** | **4.89** |
| *** Samples were not pulled for these time points for vessel #5 of Bath B.** | | | | | | | | |

### Reference Example 6

### Active Layered Core Formulation Dissolution (% Released)

Formulations A-D were tested in SGF for the total amount of naltrexone HCl released over time. The results are set forth in Figure 1 and Table 6 below:

**Table 6**

| **Filler Type** | **0** | **15** | **30** | **45** | **60** | **120** |
|---|---|---|---|---|---|---|
| Formulation A | 0 | 89 | 93 | 95 | 97 | 100 |
| Formulation B | 0 | 73 | 81 | 86 | 90 | 97 |
| Formulation C | 0 | 88 | 92 | 93 | 95 | 99 |
| Formulation D | 0 | 86 | 93 | 96 | 98 | 101 |

### Reference Example 7

### Syringability of Active Layered Core Formulation

A. Materials
Scintillation vial (hand shake for 1∼20 min)
1 cc Insulin syringe 28 ½ gauge needle
B. One active layered core of Example 3 was placed in 1 mL of water. The syringability was tested after specified time periods. The results are set forth in Table 7A below:

**Table 7A**

| Time | Syringability |
|---|---|
| 1∼5 min | slow to pull up |
| 10 min | slightly viscous, still syringable |
| 20 min | more viscous, still syringable |

C. 20 active layered cores of Example 3 were placed in 5 mL of water. The syringability was tested after specified time periods. The results are set forth in Table 7B below:

**Table 7B**

| Time | Syringability |
|---|---|
| 1 min | slow to draw up |
| 3 min | same as 1 min |
| 5 min | foamy, difficult to syringe |
| 10 min | very difficult to pull up |

### Reference Example 8

### Syringability of Multiple Active Layered Core Formulations

Multiple tablet formulations prepared in accordance with the present invention were crushed, diluted with 2 ml of water and heated. Variations were as follows:

Formulation 8A was 1 dose (20 tablets) crushed between 2 tablespoons, diluted with 2 ml of water, and heated for 2 minutes (while stirring with a needle) with a butane lighter.

Formulation 8B was a double dose (40 tablets) crushed with a mortar and pestle, transferred to a spoon and heated for 2 minutes (while stirring with a needle) with a butane lighter.

Formulation 8C was a double dose crushed with a mortar and pestle, transferred to a spoon and heated for 2 minutes dry. 2 ml of water was then added and the mixture was stirred with a needle.

Formulations 8D to 8H were all performed using 40 tablets placed in 20 ml scintillation vials. 2 ml of water was added to each and then they each were shaken for 1, 3, 5, 12 & 15 minutes.

Formulation 8D was shaken by hand. Formulations 8E to 8G were mixed on a vortex. Formulation 8H was placed on a wrist action shaker. The liquid was removed using a 1 ml insulin syringe and then placed in a 25 ml volumetric flask and filled with SGF.

Results are set forth in Table 8 below:

**Table 8**

| Formulation | Volume drawn in ml | Assay results in mg of drug | % of theoretical | Appearance & observations |
|---|---|---|---|---|
| 8A | 0.8 | 1.7 | 34.2 | Difficult to crush using 2 spoons. Took 2 minutes to draw up. |
| 8B | 0.15 | 0.6 | 6.4 | Purple in color, much more viscous. Complete glob after 3 minutes. Unable to effectively draw up. |
| 8C | 0.7 | 2.6 | 25.8 | Thin, but not easily syringed. Cotton used after a few minutes. Took 6 minutes to draw up. |
| 8D | 2 | 8.3 | 82.8 | Liquid was grayish pink, easily drawn up. |
| 8E | 1.5 | 7.3 | 72.7 | Liquid was grayish pink, slight issue with tablets drawn to needle. |
| 8F | 1.35 | 6.5 | 65.3 | Liquid was grayish pink, more difficult to avoid tablets on tip of needle. |
| 8G | 1.2 | 5.2 | 52.3 | Liquid was grayish pink, tablets were blocking syringe, they were removed from liquid at top of vial. Difficulty drawing up. |
| 8H | 1.4 | 5.8 | 58.4 | Liquid was grayish pink. Similar to 8G. |

### Reference Example 9

### Preparation of Controlled Release Formulation (Prophetic)

The active layered cores of Example 3 are coated with a controlled release coating as follows:
1. An aqueous dispersion of a neutral copolymer based on ethyl acrylate and methyl methacrylate (Eudragit NE 40 D) is coated onto the subsrate with a Vector VFC-3 Fluid Bed Processor, 4 liter chamber fitted with a Wurster Column.
2. The processing parameters are as follows: The product temperature set point is set at ambient temperature; the product temperature is ambient temperature; the exhaust temperature is approximately 45° to about 55° C; the spray rate is approximately 4 grams per minute and the process air volume is approximately 70 cfm.
3. The Eudragit NE 40 D dispersion is coated onto the Active layered cores of Example 3 to a weight gain of 20%.

### Reference Example 10

### Preparation of Controlled Release Formulation (Prophetic)

The active layered cores of Example 3 are coated with a controlled release coating as follows:
1. An aqueous dispersion of a ethylcellulose (Surelease) is coated onto the subsrate with a Vector VFC-3 Fluid Bed Processor, 4 liter chamber fitted with a Wurster Column.
2. The processing parameters are as follows: The product temperature set point is set at ambient temperature; the product temperature is ambient temperature; the exhaust temperature is approximately 45° to about 55° C; the spray rate is approximately 4 grams per minute and the process air volume is approximately 70 cfm.
3. The Eudragit NE 40 D dispersion is coated onto the Active layered cores of Example 3 to a weight gain of 20%.

## Claims

1. An oral dosage form comprising from 2 to 75 particles, each particle consisting of:
(i) a core comprising a gelling agent in the form of a compressed tablet;
(ii) an active layer comprising a drug susceptible to abuse encompassing the core; and
(iii) optionally an outer coating directly coated onto the final layered compressed core;
wherein the dosage form releases at least 85% of the drug within 45 minutes as measured by in-vitro dissolution in a USP Apparatus 1 (basket) at 100 rpm in 900 ml simulated gastric fluid without enzymes (SGF) at 37° C; and
wherein the viscosity of the dosage form mixed with from 0.5 to 10 ml of an aqueous liquid is at least 10 mPa s (10 cP).

2. The dosage form of claim 1, wherein the ratio of gelling agent to drug is from 5:1 to 1:5.

3. The oral dosage form of any of claims 1-2, wherein the gelling agent is selected from the group consisting of sugars, sugar derived alcohols, cellulose derivatives, gums, polymers, and mixtures thereof.

4. The oral dosage form of any of claims 1-3, wherein the gelling agent is selected from the group consisting of polyethylene oxide, hydroxypropylcellulose, hydroxyethylcellulose, hydroxypropylmethylcellulose, and mixtures thereof.

5. The oral dosage form of any of claims 1-4, wherein the gelling agent is polyethylene oxide.

6. The dosage form of any of claims 1-5, wherein the from 2 to 75 particles comprise a therapeutically effective amount of the drug.

7. The dosage form of any of claims 1-6, wherein the drug is selected from the group consisting of an opioid agonist, a tranquilizer, a CNS depressant, a CNS stimulant, a sedative hypnotic, and mixtures thereof.

8. The dosage form of any of claims 1-7, wherein the drug is an opioid agonist.

9. The dosage form of claim 8, wherein the opioid agonist is selected from the group consisting of codeine, morphine, oxycodone, oxymorphone, hydrocodone, hydromorphone, pharmaceutically acceptable salts thereof, and mixtures thereof.

10. The dosage form of claim 9, wherein the opioid agonist is oxycodone or a pharmaceutically acceptable salt thereof.

11. The dosage form of any of claims 1-10, comprising from 10 to 50 particles.

12. The dosage form of any of claims 1-11, wherein the particles are contained within a pharmaceutically acceptable capsule.

13. The dosage form of any of claims 1-12, further comprising a diluent contained within the pharmaceutically acceptable capsule.

14. The dosage form of claim 13, wherein the diluent is a saccharide.

## Patentansprüche

1. Orale Darreichungsform, umfassend von 2 bis 75 Partikeln, jeder Partikel bestehend aus:
(i) einem Kern, umfassend ein Geliermittel in Form einer gepressten Tablette;
(ii) einer aktiven Schicht, umfassend ein Arzneimittel, das gegenüber dem den Kern umfassenden Übergriff anfällig ist; und
(iii) optional einer äußeren Beschichtung, die direkt auf den endgültig beschichteten, gepressten Kern aufgetragen wird;
wobei die Darreichungsform wenigstens 85 % des Arzneimittels innerhalb von 45 Minuten freisetzt, gemessen anhand von in-vitro Auflösung in einem USP-Apparat 1 (Korb) bei 100 U/min in 900 ml simulierter Magenflüssigkeit ohne Enzyme (SGF) bei 37 °C; und
wobei die Viskosität der Darreichungsform, die mit von 0,5 bis 10 ml einer wässrigen Flüssigkeit gemischt ist, wenigstens 10 mPa s (10 cP) beträgt.

2. Darreichungsform nach Anspruch 1, wobei das Verhältnis des Geliermittels zum Arzneimittel von 5:1 bis 1:5 beträgt.

3. Orale Darreichungsform nach einem der Ansprüche 1 - 2, wobei das Geliermittel ausgewählt ist aus der Gruppe bestehend aus Zuckern, von Zucker abgeleiteten Alkoholen, Cellulosederivaten, Gummis, Polymeren sowie Mischungen davon.

4. Orale Darreichungsform nach einem der Ansprüche 1 - 3, wobei das Geliermittel ausgewählt ist aus der Gruppe bestehend aus Polyethylenoxid, Hydroxypropylcellulose, Hydroxyethylcellulose, Hydroxypropylmethylcellulose sowie Mischungen davon.

5. Orale Darreichungsform nach einem der Ansprüche 1 - 4, wobei das Geliermittel Polyethylenoxid ist.

6. Darreichungsform nach einem der Ansprüche 1 - 5, wobei die von 2 bis 75 Partikel eine therapeutisch effektive Menge des Arzneimittels umfassen.

7. Darreichungsform nach einem der Ansprüche 1 - 6, wobei das Arzneimittel ausgewählt ist aus der Gruppe bestehend aus einem Opiat-Agonisten, einem Beruhigungsmittel, einem CNS-Sedativum, einem CNS-Stimulans, einem beruhigenden Hypnotikum sowie Mischungen davon.

8. Darreichungsform nach einem der Ansprüche 1 - 7, wobei das Arzneimittel ein Opiat-Agonist ist.

9. Darreichungsform nach Anspruch 8, wobei der Opiat-Agonist ausgewählt ist aus der Gruppe bestehend aus Codein, Morphin, Oxycodon, Oxymorphon, Hydrocodon, Hydromorphon, pharmazeutisch verträgliche Salze davon sowie Mischungen davon.

10. Darreichungsform nach Anspruch 9, wobei der Opiat-Agonist Oxycodon oder ein pharmazeutisch verträgliches Salz davon ist.

11. Darreichungsform nach einem der Ansprüche 1 - 10, umfassend von 10 bis 50 Partikeln.

12. Darreichungsform nach einem der Ansprüche 1 - 11, wobei die Partikel innerhalb einer pharmazeutisch verträglichen Kapsel enthalten sind.

13. Darreichungsform nach einem der Ansprüche 1 - 12, ferner umfassend ein Verdünnungsmittel, das innerhalb der pharmazeutisch verträglichen Kapsel enthalten ist.

14. Darreichungsform nach Anspruch 13, wobei das Verdünnungsmittel ein Saccharid ist.

## Revendications

1. Forme posologique orale comprenant 2 à 75 particules, chaque particule étant constituée des éléments suivants :
(i) un noyau comprenant un agent gélifiant sous la forme d'une pastille comprimée ;
(ii) une couche active comprenant un médicament susceptible d'usage abusif englobant le noyau ; et
(iii) éventuellement un enrobage externe recouvrant directement le noyau comprimé à couche finale ;
dans laquelle la forme posologique libère au moins 85 % du médicament en l'espace de 45 minutes d'après la mesure effectuée *in vitro* dans un appareil de dissolution USP (corbeille) 1 à une vitesse de 100 tpm dans 900 ml de liquide gastrique simulé (SGF) sans enzymes à une température de 37 °C ; et
dans laquelle la viscosité de la forme posologique mélangée à un volume de 0,5 à 10 ml d'un liquide aqueux est au moins 10 mPa.s (10 cP).

2. Forme posologique selon la revendication 1, dans laquelle le rapport agent gélifiant sur médicament est compris entre 5:1 et 1:5.

3. Forme posologique selon la revendication 1 ou 2, dans laquelle l'agent gélifiant est choisi dans le groupe constitué par les sucres, les alcools dérivés de sucres, les dérivés de cellulose, les gommes, les polymères et leurs mélanges.

4. Forme posologique orale selon l'une quelconque des revendications 1 à 3, dans laquelle l'agent gélifiant est choisi dans le groupe constitué par l'oxyde de polyéthylène, l'hydroxypropylcellulose, l'hydroxéthylcellulose, l'hydroxypropylméthylcellulose et leurs mélanges.

5. Forme posologique orale selon l'une quelconque des revendications 1 à 4, dans laquelle l'agent gélifiant est l'oxyde de polyéthylène.

6. Forme posologique selon l'une quelconque des revendications 1 à 5, dans laquelle une quantité de 2 à 75 particules comprennent une quantité thérapeutiquement efficace du médicament.

7. Forme posologique selon l'une quelconque des revendications 1 à 6, dans laquelle le médicament est choisi dans le groupe constitué par un agoniste opioïde, un tranquillisant, un dépresseur du SNC, un stimulant du SNC, un sédatif-hypnotique et leurs mélanges.

8. Forme posologique selon l'une quelconque des revendications 1 à 7, dans laquelle le médicament est un agoniste opioïde.

9. Forme posologique selon la revendication 8, dans laquelle l'agoniste opioïde est choisi dans le groupe constitué par la codéine, la morphine, l'oxycodone, l'oxymorphone, l'hydrocodone, l'hydromorphone, leurs sels pharmaceutiquement acceptables et leurs mélanges.

10. Forme posologique selon la revendication 9, dans laquelle l'agoniste opioïde est l'oxycodone ou un sel pharmaceutiquement acceptable de celui-ci.

11. Forme posologique selon l'une quelconque des revendications 1 à 10, comprenant 10 à 50 particules.

12. Forme posologique selon l'une quelconque des revendications 1 à 11, dans laquelle les particules sont contenues dans une capsule pharmaceutiquement acceptable.

13. Forme posologique selon l'une quelconque des revendications 1 à 12, comprenant en outre un diluant contenu dans la capsule pharmaceutiquement acceptable.

14. Forme posologique selon la revendication 13, dans laquelle le diluant est un saccharide.
